# EUROPÄISCHE PATENTSCHRIFT

(11) **EP 2 581 059 B1**
(45) Veröffentlichungstag und Bekanntmachung des Hinweises auf die Patenterteilung: **22.03.2017**
(21) Anmeldenummer: 11184918.8
(22) Anmeldetag: 12.10.2011
(51) Int. Cl.: A61B 18/14, A61B 17/29

(54) **Chirurgisches Instrument mit erhöhter Verlässlichkeit**
Surgical instrument with improved reliability
Instrument chirurgical doté d'une fiabilité améliorée

(43) Veröffentlichungstag der Anmeldung: 17.04.2013
(73) Patentinhaber: Erbe Elektromedizin GmbH, 72072 Tübingen (DE)
(72) Erfinder: Baur, Thomas, 72108 Rottenburg (DE); Hiller, Jürgen, 72581 Dettingen (DE); Rydzewski, Viktoria, 72622 Nürtingen (DE)
(74) Vertreter: Rüger, Barthelt & Abel

(56) Entgegenhaltungen:
- EP-A1- 2 095 778
- DE-U1- 8 910 462
- US-A- 5 766 167
- US-A- 5 810 876

## Beschreibung

Die Erfindung betrifft ein chirurgisches Instrument, insbesondere ein endoskopisches Instrument für ein- oder mehrmaligen Gebrauch.

Chirurgische Instrumente werden beispielsweise zum Abklemmen und Verschließen, sowie gegebenenfalls auch zum Durchtrennen von abgeklemmten und verschlossenen Blutgefäßen eingesetzt. Solche Instrumente gehen beispielsweise aus der DE 298 04 860 U1, der US 5,984,939, der US2010/0234687, der WO 2010/009525 A1, der DE 10 2010 040 667 A1, der WO 2004/091377 A2, der WO 97/41783, der US 2009/017147 A1, der WO 2008/020964 A2 oder auch der US 5,855,590 hervor. Diesen meist für den laproskopischen oder endoskopischen Einsatz vorgesehenen Instrumenten ist gemeinsam, dass sie einen länglichen Schaft aufweisen, der sich von einem Griff weg erstreckt. An dem distalen Ende des Schafts ist ein Werkzeug angebracht, das von dem Griff aus über eine Betätigungseinrichtung bewegt werden kann. Ein Übertragungsmittel stellt eine Getriebeverbindung zwischen der Betätigungseinrichtung und dem Werkzeug her. Das Übertragungsmittel ist zum Beispiel ein Zugdraht, der sich durch den rohrartigen Schaft erstreckt. Naturgemäß muss zischen dem Zugdraht und der Innenwand des Schafts ein gewisses Spiel vorhanden sein, um die Freigängigkeit des Zugdrahts sicherzustellen.

Chirurgische Instrumente werden in einem feuchten Milieu eingesetzt. Dringt Feuchtigkeit, beispielsweise Gewebeflüssigkeit irgendeiner Art, Spülflüssigkeit oder dergleichen, in ein chirurgisches Instrument ein, kann dies dort zu elektrischen oder sonstigen Fehlfunktionen führen. Dies gilt mindestens bei chirurgischen Instrumenten für einmaligen Gebrauch. Bei chirurgischen Instrumenten für mehrmaligen Gebrauch kommt hinzu, dass bei den zwischen verschledenen Benutzungen nötigen Sterilisierungsvorgängen Flüssigkeit in das Gehäuse eindringen und dort wiederum Fehler verursachen könnte.

Aus der US-A-5766167 ist ein chirurgisches Instrument mit einem Schaft bekannt, der als längliches Rohr ausgebildet ist. An seinem distalen Ende trägt er eine scherenartige Anordnung, die durch eine Zugstange betätigt wird. Die Zugstange besteht aus Kunststoff und weist in ihrem Inneren einen elektrisch leitenden Draht auf. Die Zugstange ist durch eine an der endseitigen Stirnfläche des Schafts angeordnete Scheibendichtung geführt, um ein Eindringen von Fluid in den Schafft zu verhindern.

Bei dem aus der EP 2 095 778 A1 bekannten Instrument, sind Zugelemente durch eine scheibenförmige, im Schaft angeordnete Dichtung gefuhrt.

Ein weiteres chirurgisches Instrument ist aus der US-A-5,810,876 bekannt, das ebenfalls einen länglichen Schaft aufweist, der an seinem proximalen Ende einen Griff und an seinem distalen Ende ein Werkzeug zur Vornahme operativer Eingriffe an einem menschlichen Körper aufweist. Dieses Werkzeug ist wiederum als Schere oder Zange ausgebildet, die über einen Zugdraht betätigt wird. Dieser ist zangenseitig mit einem Stift verbunden, der in einer Dichtung geführt ist. Die Dichtung kann ein O-Ring, eine glockenförmige Gummidichtung, eine Gummischeibe oder ein Gummizylinder sein. Sie ist letztlich Teil des Werkzeugs.

Eine weitere Dichtung kann griffseitig angeordnet sein. Griffseitig läuft der Betätigungsdraht in einer Hülse, die das proximale Ende des Schafts übergreift und ihrerseits endseitig mit einer Klemmschraube gefasst ist. Die Hülse ist durch einen O-Ring oder eine glockenförmige Dichtung gegen den Griff abgedichtet. Alternativ zu den genannten Dichtungen wird auch die Füllung des Lumens, durch das sich die Hülse erstreckt, mit Dichtungsmasse wie bspw. Silikon vorgeschlagen.

Unabhängig davon erstreckt sich in allen Ausführungsformen der Zugdraht durch den offenen Innenraum des Schafts.

Die DE-A-891042 offenbart ein chirurgisches Instrument mit einem länglichen Schaft, durch den sich eine manuell verschiebbare Betätigungsstange erstreckt. Zur Abdichtung derselben ist griffseitig an dem proximalen Ende des Schafts eine hohlzylindrische Gummihülse vorgesehen.

Es ist Aufgabe der Erfindung, ein verbessertes chirurgisches Instrument zu schaffen.

Diese Aufgabe wird mit dem Instrument nach Anspruch 1 gelöst:
Das erfindungsgemäße Instrument kann ein Einweginstrument für den einmaligen Gebrauch wie auch ein sterilisierbares für den mehrmaligen Gebrauch geeignetes Instrument sein. Es weist einen länglichen Schaft auf, an dessen einen Ende ein Werkzeug, welches beispielsweise ein bewegliches Teil umfasst und an dessen anderen Ende ein Griff mit einer Betätigungseinrichtung für das Werkzeug vorgesehen ist. Durch den länglichen Schaft erstreckt sich mindestens ein Übertragungsmittel, das eine Bewegung der Betätigungseinrichtung auf das Werkzeug überträgt. In dem Schaft ist ein Dichtungskörper angeordnet, der als gesondert gefertigtes Dichtelement in den Schaft eingesetzt sein oder auch dort durch Urformen ausgebildet sein kann.

Unter gesonderter Fertigung wird dabei jede Herstellung in und außerhalb des Betriebs des Herstellers des Instruments verstanden. Es kann sich um handelsübliche oder speziell angefertigte Dichtelemente handeln.

Unter Urformen wird jede Erzeugung des Dichtelementes aus einer zunächst formlosen Masse verstanden. Diese wird in fließfähigem zum Beispiel breiigem oder zähflüssigem oder plastisch-zähflüssigem Zustand in den Schaft eingebracht und verschließt den dort vorhandenen Durchgang. Vorzugsweise sind dabei das oder die Übertragungsmittel bereits in dem Schaft angeordnet. Nach dem Eindringen der formlosen Masse bildet sich aus dieser das Dichtelement, indem die formlose Masse einen festen, gegebenenfalls elastischen Zustand einnimmt. Hierzu können chemische und/oder physikalische Vorgänge dienen. Beispielsweise kann die Ausbildung des Dichtelements durch Abkühlen einer durch Wärme verflüssigten Masse, durch Aushärten bspw. mittels chemischer Reaktionen, z.B. Vernetzung, durch Trocknen oder durch sonstige Vorgänge erfolgen. Die chemische Vernetzung kann durch chemische Beschleuniger ausgelöst und bewirkt werden. Es können schnell bindende Zweikomponentenmischungen genutzt werden, die kurz vor dem oder während des Einbringens gemischt werden. Es können physikalische Vernetzungsauslöser, z.B. Strahlung, z.B. UV-Strahlung genutzt werden, um die Aushärtung auszulösen oder zu bewirken. Als Material für das Dichtelement können Werkstoffe mit selbstschmierenden Eigenschaften ausgewählt werden.

Das in den Schaft eingebaute oder im Schaft gebildete Dichtelement versperrt im Schaft den Durchgang von Flüssigkeit, Dampf, Gasen, Aerosolen, Stäuben, Rauch und dergleichen. Das mindestens eine Übertragungsmittel erstreckt sich dabei durch das Dichtelement. vorzugsweise ist das Übertragungsmittel in dem Dichtelement längsbeweglich angeordnet. Dies kann durch verschiedene Maßnahmen ermöglicht werden. Zum Beispiel kann das Betätigungsmittel eine relativ glatte, z.B. metallische Oberfläche aufweisen.

Ist das z.B. durch Injektion einer formlosen Masse erzeugte Dichtelement ausgehärtet und wird das Betätigungmittel erstmalig in Längsrichtung bewegt, überwinden die zwischen dem Dichtelement und dem Betätigungsmittel wirkenden Scherkräfte die zwischen dem Betätigungsmittel und dem Dichtelement vorhandenen Haftkräfte. Der evtl. vorhandene Verbund zwischen dem Übertragungsmittel und dem Dichtelement wird zumindest teilweise, vorzugsweise aber vollständig aufgetrennt. Das Dichtelement und das Übertragungsmittel verbleiben in reibschlüssiger Anlage. Weil die Kontaktfläche zwischen dem Dichtelement und dem Betätigungsmittel deutlich geringer ist als die Kontaktfläche zwischen dem Dichtelement und der inneren Wandung des Schafts, ist sichergestellt, dass bei einer ersten Betätigung das Dichtelement am Platz bleibt und die äußere Verbindung zum Schaft nicht aufgetrennt wird. Der sich zwischen dem Betätigungsmittel und dem Dichtelement bildende Spalt hat die Spaltweite Null. Dies genügt, um das Eindringen von Feuchtigkeit, Gasen oder sonstigen Partikeln zu verhindern. Etwaige an den beweglichen Übertragungsmitteln sitzende Flüssigkeits- oder Feststoffpartikel werden durch das Dichtelement von der Oberfläche des Betätigungsmittels abgestreift. Ein Verschleppen in das Gehäuse durch Mitnahme, Pumpeffekte oder dergleichen wird vermieden.

Es können im Rahmen der Erfindung weitere oder andere Maßnahnen getroffen werden, um das dicht und spaltfrei an dem Betätigungsmittel anliegende Dichtelement durch Urformen zu erzeugen. Zum Beispiel kann das Betätigungsmittel eine Antihaftbeschichtung aufweisen, zum Beispiel eine PE-Beschichtung, ein PTFE-Beschichtung, eine Silkonölschicht oder dergleichen. Als Dichtungsmasse eignen sich insbesondere nicht schrumpfende Massen wie bspw. Silikorgummi vom Typ Alkoxy-Silicon. Es werden zur Erstellung des Dichtelements vorzugsweise wasserabweisende (hydrophobe) Stoffe bevorzugt. Diese verhindern wirksam das Eindringen von Feuchtigkeit in einen wie auch immer gearteten Spalt zwischen dem Dichtelement und dem Betätigungsmittel. Die oben erwähnte Abstreifwirkung ist verlässlich gegeben. Es wird ein Einschleppen von 'Feuchtigkeit durch die Längsbewegung des Betätigungsmittels in den Innenraum des Griffteils verhindert.

Das Dichtelement kann kompakt (porenfrei) ausgebildet. sein. Bedarfsweise kann es auch ein gewisses Porenvolumen aufweisen, wobei eine geschlossenzellige Struktur bevorzugt wird. Es sind jedoch auch offenzellige Strukturen nutzbar. Ein Beispiel sind Dichtelemente aus einem offenzellig geschäumten Werkstoff. Auch ein solcher verhindert den Durchtritt von Flüssigkeiten zumindest für eine bestimmte Zeit. Dies erst recht, wenn der Schaum wasserabweisend ausgerüstet ist. Auch andere Stoffe, z.B. Filz, insbesondere Filz, der quellfähige Fasern enthält kann eingesetzt werden. Diese Konzepte sind insbesondere zur Anwendung in Einweggeräten geeignet.

Das Übertragungsmittel ist vorzugsweise ein Draht oder eine dünne Stange aus Metall oder Kunststoff. Das Übertragungsmittel ist mindestens zur Übertragung von Zugkräften geeignet. Es kann auch zur Übertragung von Schubkräften eingerichtet sein. Ist das Übertragungsmittel aus Metall oder einen sonstigen elektrisch leitenden Werkstoff ausgebildet, kann es nicht nur mechanische Bewegungen, sondern auch elektrische Energie übertragen. Ist das Übertragungsmittel ein Draht oder eine dünne Stange aus Kunststoff wird vorzugsweise ein Kunststoff gewählt, der sich mit dem Material des Dichtelements, z.B. Silikon, nicht vereinigt oder an diesem wenig oder nicht haftet.

Ist das Übertragungsmittel aus Metall, ist es vorzugsweise blank. Dies bedeutet, dass die Metalloberfläche glatt und frei von einer weiteren Beschichtung, wie bspw. einer Isolierung, ist. Allerdings kann, wenn dies gewünscht wird, auf dem blanken Draht auf ganzer Länge oder einem Teil desselben auch eine Isolierung oder eine Antihaftschicht angebracht sein. Die Isolierung kann sich auf Teile erstrecken, die nicht mit dem Dichtelement in Berührung stehen und/oder auch auf Teile, die sich durch das Dichtelement hindurch erstrecken.

Das Instrument kann ein Werkzeug mit einem oder mehreren beweglichen Teilen umfassen. Ein beweglich angeordnetes Teil kann eine lineare Bewegung, beispielsweise in Schaftlängsrichtung, oder auch eine axiale Bewegung, um einen Drehpunkt herum, ausführen. Die beweglichen Teile können einem oder mehreren Übertragungsmitteln zugeordnet sein, die sich durch den Schaft und das Dichtelement erstrecken. Vorzugsweise sind die Übertragungsmittel unabhängig voneinander und somit relativ zueinander beweglich. Die sich durch das Dichtelement erstreckenden Abschnitte der Übertragungsmittel sind vorzugsweise gerade ausgebildet und in Bewegungsrichtung orientiert. So wird eine ungehinderte Bewegung des durch das Dichtelement gleitenden Übertragungsmittels sichergestellt.

Das Dichtelement ist vorzugsweise aus einem nicht schrumpfenden Werkstoff ausgebildet. Damit kann sichergestellt werden, dass es den gesamten Querschnitt des Schafts verschließt und somit eine gute Dichtigkeit garantiert. Das Dichtelement kann, wie erwähnt, aus einem hydrophoben Material gebildet sein. Das Betätigungsmittel kann ebenfalls aus einem hydrophoben Material bestehen, beispielsweise einem Kunststoff, oder mit einem hydrophoben Material beschichtet sein. Dies gilt insbesondere für den sich durch das Dichtelement erstreckenden Abschnitt des Betätigungsmittels. Eine solche Beschichtung kann ein Eindringen von Wasser in den Innenraum des Geräts auch dann verhindern, wenn gewisse Druckdifferenzen vorhanden sind, wie sie bspw. beim Sterilisieren eines chirurgischen Instruments auftreten könnten. Auch bei der Anwendung von Einweggeräten können erhebliche Druckdifferenzen z.B. bei der Insufflation auftreten.

Das Dichtelement weist vorzugsweise eine Länge auf, die deutlich geringer ist als die Länge des Schafts, wobei sie vorzugsweise größer ist als der Durchmesser des Schafts. Auf diese Weise wird einerseits die Dichtheit garantiert und andererseits eine Leichtgängigkeit des wenigstens einen Übertragungsmittels erhalten. Bei Nutzung von Dichtmaterialien, die mit den verwendeten Übertragungsmitteln einen geringen Reibbeiwert festlegen und z.B. selbstschmierende Eigenschaften haben, kann das Dichtelement auch mehr als die Hälfte der Länge des Schaft, z.B. seine gesamte Länge einnehmen.

Es ist prinzipiell möglich, das Dichtelement in dem Schaft verschiebbar anzuordnen. Bei solch einer Ausführungsform kann dann das Übertragungsmittel stoffschlüssig und/oder reibschlüssig und/ oder formschlüssig mit dem Dichtelement verbunden sein. Vorzugsweise ist das Dichtelement jedoch in dem Schaft stoffschlüssig und/oder reibschlüssig und/ oder formschlüssig gehalten. Damit wird sichergestellt, dass es auch nach längerem Gebrauch nicht wandert, sondern am Ort bleibt, während das Betätigungsmittel hin- und hergehend durch das Dichtelement gleiten kann.

Weitere Einzelheiten vorteilhafter Ausführungsformen der Erfindung sind Gegenstand der Zeichnung, der Beschreibung oder von Ansprüchen. Es zeigen:
Figur 1 das erfindungsgemäße Instrument, in einer perspektivischen Gesamtansicht,
Figur 2 das Instrument nach Figur 1, in einer schematischen Prinzipdarstellung,
Figur 3 das Instrument nach Figur 1, mit abgenommener Kappe,
Figur 4 das Instrument nach Figur 1, in ausschnittsweiser perspektivischer Darstellung, geschnitten im Bereich eines Dichtelements, und
Figur 5 eine gesonderte Darstellung des Schafts, der sich durch ihn erstreckenden Betätigungsmittel und des Dichtelements, in schematisierter, längs geschnittener Darstellung.

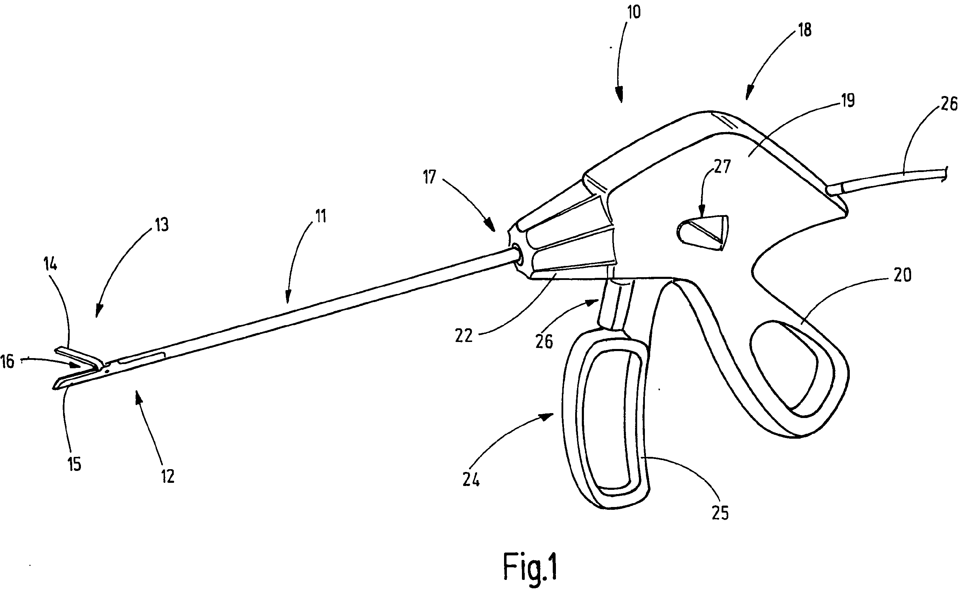

In Figur 1 ist ein chirurgisches Instrument 10 veranschaulicht, wie es bspw. bei endoskopischen oder laproskopischen Operationen aber auch bei Operationen mit offenem Operationsfeld eingesetzt werden kann. Das in Figur 1 dargestellte Instrument 10 stellt lediglich ein Beispiel für ähnlich geartete Instrumente dar, an denen die Erfindung Anwendung finden kann. Das Instrument 10 dient dem Abklemmen und Verschließen von Blutgefäßen. Bedarfsweise kann es außerdem zum Durchtrennen eines verschlossenen Blutgefäßes eingerichtet sein.

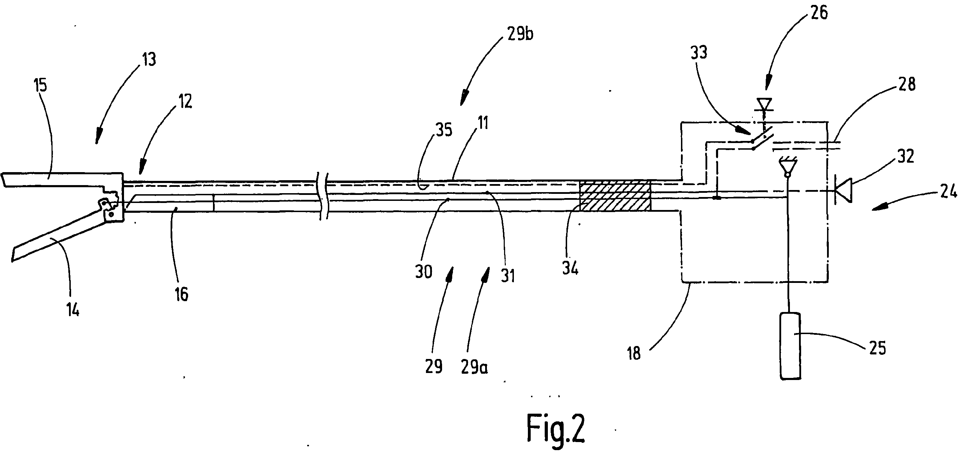

Das Instrument 10 weist einen länglichen, vorzugsweise geraden Schaft 11 auf, der vorzugsweise einen Durchmesser von bspw. lediglich wenigen Millimetern und eine Länge von einigen Dezimetern aufweisen kann. Figur 1 ist insoweit nicht maßstäblich. Der Schaft 11 trägt an seinem distalen Ende 12 ein Werkzeug 13, das mit biologischem Gewebe, hier bspw. einem Gefäß, in Wechselwirkung gebracht wird. Das Werkzeug 13 umfasst mindestens einen beweglichen Teil 14, der z.B. schwenkbeweglich an einem anderen Teil 15 gelagert sein kann. Bedarfsweise können auch beide Teile 14, 15 beweglich, z.B. schwenkbeweglich gelagert sein. Zu dem Werkzeug 13 kann außerdem ein Messer 16 gehören, das besser aus Figur 2 ersichtlich und z.B. längs verschiebbar gelagert ist. Das Messer 16, das normalerweise in einer zurückgezogenen, schematisch in Figur 2 angedeuteten Position steht, kann z.B. dazu genutzt werden, ein zwischen den Teilen 14, 15 gehaltenes, z.B. durch Koagulation geschlossenes Gefäß zu durchtrennen, indem es längs vorgeschoben wird. Die Teile 14, 15 können dazu mit entsprechenden Aussparungen oder Schlitzen versehen sein.

Der Schaft 11 weist außerdem ein proximales Ende 17 auf, das an einem Griff 18 gehalten ist. Der Griff 18 kann als Gehäuse ausgebildet sein und einen oberen Gehäuseteil 19 sowie einen unteren Griffteil 20 aufweisen. Der Gehäuseteil 19 und/oder der Griffteil 20 sind vorzugsweise hohl ausgebildet.

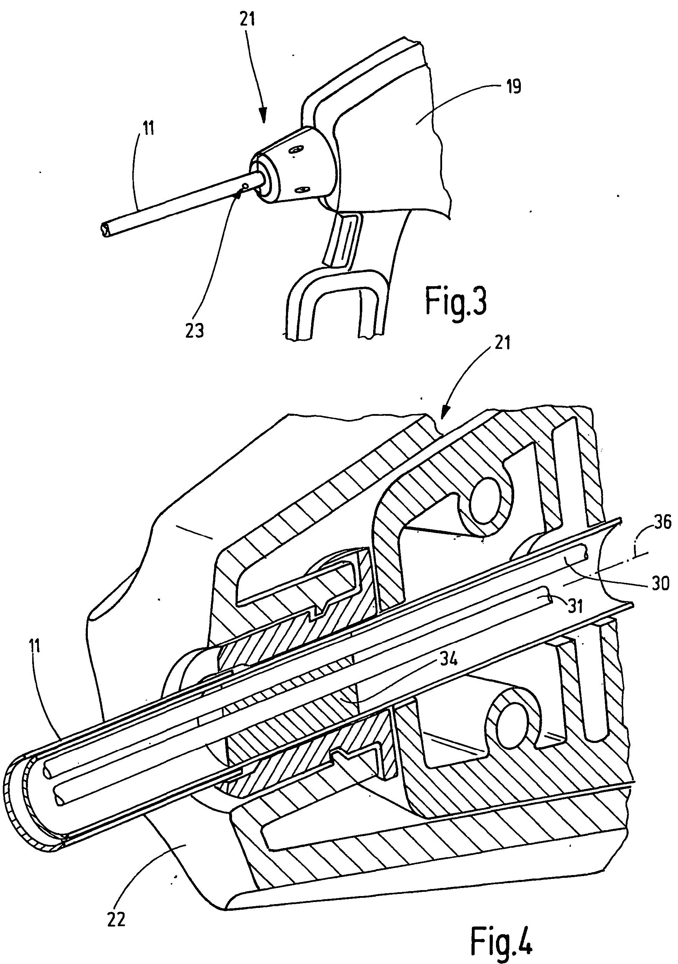

Zur Verbindung des proximalen Endes 17 des Schafts 11 mit dem Gehäuseteil 19 dient eine Kupplungs- oder Verbindungseinrichtung 21, wie sie aus den Figuren 3 und 4 hervorgeht. Der Gehäuseteil 19 weist an seinem in Schaft 11 zugewandten Ende einen konischen Ansatz auf, der normalerweise von einem Drehgriff 22 übergriffen wird, Dieser Drehgriff 22 dient dazu, den Schaft 11 beim Einsatz in eine gewünschte Drehstellung um seine Längsachse zu drehen. Er kann dazu mit einem entsprechenden Loch 23 in Eingriff stehen, das in dem proximalen Ende 17 des Schafts 11 ausgebildet ist.

An dem Griff 18, insbesondere an dem Gehäuseteil 19 kann eine Betätigungseinrichtung 24 für das Werkzeug angeordnet sein. Diese Betätigungseinrichtung 24 kann bspw. einen schwenkbar gelagerten Handhebel 25 umfassen, der zu dem Griffteil 20 hin und von diesem weg schwenkbar ist. Außerdem kann die Betätigungseinrichtung 24 weiter Betätigungsmittel umfassen, wie bspw. einen Auslöser 26 und/oder einen weiteren Betätigungshebel 27. Außerdem erstreckt sich von dem Gehäuse 18 eine Leitung 28 weg, die als elektrisches Kabel ausgebildet sein kann. Dieses führt z.B. zu einem speisenden medizinischen Gerät, welches das Werkzeug 13 bei Bedarf mit elektrischer Energie versorgt.

Wie insbesondere aus der äußerst schematischen Figur 2 hervorgeht, ist das Werkzeug 13 mit der Betätigungseinrichtung 24 verbunden. Dazu erstreckt sich z.B. mindestens ein Übertragungsmittel 29 längs durch den hohlen Schaft 11. Das Übertragungsmittel 29 kann bspw. ein aus Kunststoff oder Metall bestehender Draht 30 sein, der z.B. vorwiegend als Zugmittel dient. Er kann mit einem Ende an dem Teil 14 des Werkzeugs 13 und mit seinem anderen Ende direkt oder über ein Getriebe mit dem Handhebel 25 verbunden sein.

Durch den hohlen Schaft 11 können sich weitere Übertragungsmittel 29a, 29b erstrecken, bspw. in Gestalt eines etwas dickeren Drahts oder einer Stange 31, die Schubkräfte übertragen kann. Ein Ende der Stange 31 kann mit dem Messer 16 verbunden sein. Ein anderes Ende der Stange 31 kann mit einem eigenen Betätigungsmittel 32 oder auch einer Sperr- und Kupplungseinrichtung verbunden sein, die z.B. von der Betätigungseinrichtung 24 gesteuert ist. Mittels der Sperr- und Kupplungseinrichtung kann z.B. das Messer 16 an den Handhebel 25 angeschlossen werden, um von diesem betätigt zu werden. Die Sperr- und Kupplungseinrichtung kann von dem Hebel 27 gesteuert werden.

Der Auslöser 26 kann dazu dienen, den Teil 15 des Werkzeugs 13 mit einem Pol einer Spannungsquelle, vorzugsweise einer HF-Spannungsquelle, zu verbinden. Der Draht 30 kann als elektrischer Leiter dienen und den Teil 14 bedarfsweise mit dem anderen Pol der elektrischen Quelle verbinden. Der ein- oder zweipolige, von dem Auslöser 26 betätigte Schalter 33 kann die Verbindung zwischen der Leitung 28 und den Teilen 14, 15 des Werkzeugs 13 herstellen oder unterbrechen.

In dem Schaft 11 ist ein Dichtungselement 34 angeordnet, das den längs durch den Schaft 11 führenden Durchgang versperrt und durch das sich das Übertragungsmittel 29, 29a, 29b, d.h. im Einzelnen der Draht 30, die Stange 31 und gegebenenfalls auch die in Figur 2 gestrichelt dargestellte elektrische Leitung 35 erstrecken. Die Leitung 35 ist ein ruhendes Organ, das z.B. lediglich den Schalter 33 mit dem Teil 15 des Werkzeugs 13 verbindet. Die Leitung 35 ist bspw. ein isolierter Draht. Hingegen ist das Übertragungsmittel 29 oder 29a, also bspw. der Draht 30 oder die Stange 31, jeweils längsbeweglich angeordnet. Sie erstrecken sich parallel zu der Schaftmittelachse 36 und werden in dieser Richtung hin- und hergehend bewegt. Sie sind somit in dem Dichtelement 34 beweglich angeordnet.

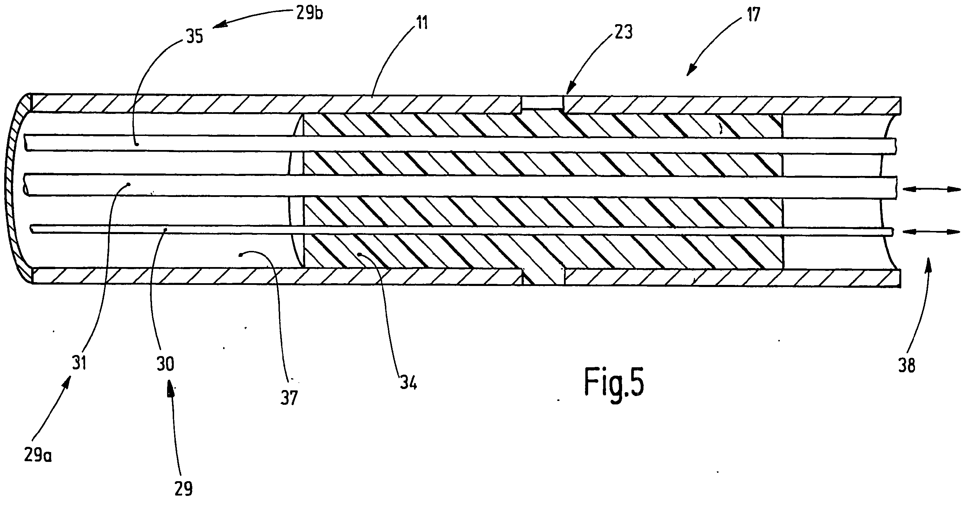

Zur weiteren Erläuterung wird auf Figur 5 verwiesen. Diese zeigt einen Abschnitt des Schafts 11, der dem Griff 18, d.h. dem proximalen Ende 17 nahe ist. In einer beispielhaften Ausführung enthält dieser Teil des Schafts 11 das Dichtelement 34, durch das sich die Übertragungsmittel 29, 29a, 29b, z.B. in Gestalt des Drahts 30 der Stange 31 und der Leitung 35 erstrecken. Die Anordnung des Dichtelements 34 am proximalen Ende des Schafts 11 minimiert die Knickneigung der Übertragungsmittel 29, wenn dieser in Richtung auf das Werkzeug 13 hin geschoben wird. Es kann jedoch auch vorteilhaft und gegebenfalls zu bevorzugen sein, das Dichtelement 34 an anderer Stelle, z.B. an dem distalen Ende 12 oder einer Stelle zwischen dem distalen Ende 12 und dem proximalen Ende 17 des Schafts 11 anzuordnen.

Das Dichtelement 34 besteht vorzugsweise aus einem elastischen wenig oder nicht schrumpfenden Kunststoff, wie bspw. einem vernetzten Silikon. Insbesondere werden hierzu aus der Fülle der verfügbaren Silikonwerkstoffe solche ausgewählt, die eine geringe Schrumpfung oder Quellung aufweisen und insbesondere wasserabweisend sind. Das Dichtelement 34 kann einen porenfreien kompakten Körper aufweisen. Es können jedoch auch andere Werkstoffe gewählt werden. Z.B. kann das Dichtelement ein Porenvolumen haben und geschlossenzellig geschäumt sein, um eine erhöhte Elastizität zu bieten. Es können auch andere Materialien, wie offenzellige Schäume oder Filze zur Anwendung kommen. Diese können bedarfsweise in Wasser auch quellfähig sein, um bei Wasserzutritt zu quellen und abzudichten.

Das Dichtelement 34 kann gesondert gefertigt und als Element bei der Montage des Instruments 10 in den Schaft eingebaut werden. Vorzugsweise wird es dabei an der Innenwand des Schafts z.B. formschlüssig, reibschlüssig oder stoffschlüssig gesichert. Die sich durch das Dichtelement erstreckenden Übertragungsmittel 29, 29a, 29b erstrecken sich vorzugsweise spaltfrei oder wenigstens im Wesentlichen spaltfrei durch das Dichtelement 34, so dass sie in Längsrichtung 36 leichtgängig hin und her bewegt werden können.

Bei einer anderen Ausführungsform ist das Dichtelement 34 am Einbauort durch Urformen hergestellt worden. Dazu wurden in dem Schaft 11 zunächst das oder die Übertragungsmittel 29 angeordnet und danach durch eine geeignete Öffnung, bspw. das Loch 23, das noch nicht ausgehärtete Material des Dichtelements 34 in dem Innenraum des Schafts 11 eingespritzt. Es füllt den Durchgang aus und umhüllt die Übertragungsmittel 29. Vorzugsweise bildet sich dabei eine stoffschlüssige Haftung zwischen dem Dichtelement 34 und der inneren Wand 37 des Schafts 11. Außerdem kann das Aushärten der Dichtelemente 34 mit ein oder mehreren Strukturen des Schafts 11 in formschlüssigem Eingriff kommen. Beispielsweise können sich ein oder mehrere Vorsprünge des Dichtelements 34 in ein oder mehrere Löcher 23 erstrecken und darin aushärten. Es kann sich eine formschlüssige Verzahnung zwischen dem Dichtelement 34 in dem Schaft 11 ausbilden, so dass das Dichtelement 34 in der Längsposition in dem Schaft 11 gesichert wird, in der es erzeugt worden ist. Zur Aushärtung des Materials des Dichtelements 34 kann je nach verwendetem Materialtyp jeweils eine geeignete Maßnahme genutzt werden, wie sie bereits erwähnt wurden.

Nach den vorstehend erläuterten Prinzipien könnten in dem Schaft 11 ein oder auch mehrere Dichtelemente 34 angeordnet und/oder ausgebildet werden. Jedes der Dichtelement kann an dem distalen Ende 12, an dem proximalen Ende 17 oder dazwischen angeordnet sein. Die Dichtelement 34 können mit oder ohne Abstand zueinander angeordnet sein.

Spätestens wenn die Übertragungsmittel 29 in Richtung der Pfeile 38 erstmalig bewegt werden, löst sich eine etwaige oberflächliche Haftung zwischen dem Übertragungsmiztel 29 und dem Dichtelement 34 durch die entstehende Konzentraticn der Scherkräfte auf. Von da an bildet das Dichtelement 34 durch die spaltfreie Anlage an der Oberfläche der Übertragungsmittel 29, beispielsweise des Drahts 30 und/oder der Stange 31 eine wirksame Sperre, insbesondere geger. wässrige Fluide aber auch Dämpfe, Gase, Stäube, Rauch oder dergleichen. Das Dichtelement 34 dichtet somit den sonst von dem Werkzeug 13 in den Innenraum des Gehäuses des Griffs 18 führenden Durchgang wirksam ab.

Ein chirurgisches Instrument 10 mit einem langen Schaft 11 weist in dem Schaft 11 ein Dichtelement 34 auf, das durch Urformen am Einbauort erzeugt worden ist. Vorzugsweise wird das Dicatelement 34 hergestellt, indem in den Schaft 11 zunächst etwaige ein oder mehrere Betätigungsmittel 29 montiert und ortsfest gehalten werden, wonach ein aushärtbarer Kunststoff, aus dem das Dichtelement 34 entstehen soll, so in den Schaft 11 injiziert wird, dass das Material das Betätigungselement 11 umhüllt und den Durchgang des Schafts 11 auf einer Länge versperrt, die vorzugsweise größer ist als der Innendurchmesser und kürzer als seine Länge. vorzugsweise wird die aushärtbare Masse in einer Länge von einem oder wenigen Zentimetern in den z.B. mehrere Dezimeter langen Schaft injiziert, dessen Innendurchmesser vorzugsweise allenfalls wenige Millimeter beträgt.

### Bezugszeichenliste:

- 10: Instrument
- 11: Schaft
- 12: distales Ende des Schafts 11
- 13: Werkzeug
- 14: schwenkbeweglicher Teil des Werkzeugs 13
- 15: fester Teil des Werkzeugs 13
- 16: Messer
- 17: proximales Ende des Schafts 11
- 18: Griff
- 19: Gehäuseteil
- 20: Griffteil
- 21: Kupplungs- und Verbindungseinrichtung
- 22: Drehgriff
- 23: Loch
- 24: Betätigungseinrichtung
- 25: Handhebel
- 26: Auslöser
- 27: Betätigungshebel
- 28: Leitung
- 29, 29a, 29b: Übertragungsmittel
- 30: Draht
- 31: Stange
- 32: Betätigungsmittel
- 33: Schalter
- 34: Dichtelement
- 35: Leitung
- 36: Schaftmittelachse
- 37: Wandung
- 38: Pfeile

## Patentansprüche

1. Chirurgisches Instrument (10), insbesondere endoskopisches Instrument,
mit einem länglichen Schaft (11), der ein Rohr ist, das einen Innenraum aufweist, und der ein proximales Ende (17) und ein distales Ende (12) aufweist,
mit mindestens einem Werkzeug (13), das an dem distalen Ende (12) angeordnet ist,
mit einem Griff (18), der an dem proximalen Ende (17) angeordnet ist,
mit wenigstens einer Betätigungseinrichtung (24), die an dem Griff (18) angeordnet ist,
mit wenigstens einem Draht(30), der sich durch den Innenraum des Schafts (11) erstreckt und in diesem beweglich ist, um eine Bewegung der Betätigungseinrichtung (24) auf das Werkzeug (13) zu übertragen,
mit wenigstens einem Dichtelement (34), das in dem Innenraum des Rohrs angeordnet ist, das den Schaft (11) bildet, und den gesamten Querschnitt des Schafts (11) verschließt, so dass sich der Draht (30) längsbeweglich durch das Dichtelement (34) ersteckt
**dadurch gekennzeichnet, dass** das Dichtelement (34) in dem Schaft (11) durch Urformen auf einer Länge ausgebildet ist, die größer ist als der Innendurchmesser und kürzer als die Länge des Schafts (11).

2. Instrument nach Anspruch 1, **dadurch gekennzeichnet, dass** das Werkzeug (13) mindestens ein linear bewegliches oder schwenkbewegliches Teil (14) umfasst, das mit dem Übertragungsmittel (29, 29a) verbunden ist.

3. Instrument nach Anspruch 1 oder 2, **dadurch gekennzeichnet, dass** das Übertragungsmittel (29, 29a, 29b) ein Draht (30) oder eine dünne Stange (31) aus Metall oder Kunststoff ist.

4. Instrument nach einem der vorhergehenden Ansprüche,
**dadurch gekennzeichnet, dass** das Übertragungsmittel (29, 29a, 29b) elektrisch leitfähig ist.

5. Instrument nach einem der vorhergehenden Ansprüche,
**dadurch gekennzeichnet, dass** das Werkzeug (13) zwei oder mehrere bewegliche Teile (14, 15) aufweist, die mit zwei oder mehreren Übertragungsmitteln (29, 29a) verbunden sind, die sich durch den Schaft (11) und durch das Dichtelement (34) erstrecken, wobei die Übertragungsmittel (29, 29a) gegeneinander relativ beweglich sind.

6. Instrument nach Anspruch 1, **dadurch gekennzeichnet, dass** wenigstens der sich durch das Dichtelement (34) erstreckende Abschnitt des Übertragungsmittels (29, 29a, 29b) gerade ausgebildet ist.

7. Instrument nach einem der vorhergehenden Ansprüche,
**dadurch gekennzeichnet, dass** das Dichtelement (34) aus einem hydrophoben Material besteht.

8. Instrument nach einem der vorhergehenden Ansprüche,
**dadurch gekennzeichnet, dass** das Dichtelement (34) eine in Schaftlängsrichtung zu messende Länge aufweist, die den Durchmesser des Dichtelements (34) um ein Mehrfaches übersteigt und/oder die gleich oder geringer ist, als die Länge des Schafts (11), wobei die Schaftlängsrichtung durch eine Schaftmittelachse (36) festgelegt ist.

9. Instrument nach einem der vorhergehenden Ansprüche,
**dadurch gekennzeichnet, dass** das Dichtelement (34) an dem distalen Ende (12) des Schafts (11), an dem proximalen Ende (17) des Schafts (11) und/oder dazwischen angeordnet ist.

10. Instrument nach einem der vorhergehenden Ansprüche,
**dadurch gekennzeichnet, dass** das Dichtelement (34) in dem Schaft (11) gegen den Schaft (11) unverschiebbar gehalten ist.

11. Instrument nach einem der vorhergehenden Ansprüche,
**dadurch gekennzeichnet, dass** das Dichtelement (34) in dem Schaft (11) kraftschlüssig, reibschlüssig, stoffschlüssig und/oder formschlüssig gehalten ist.

## Claims

1. Surgical instrument (10), in particular endoscopic instrument,
with a longitudinal shaft (11) which is a tube having an interior, a proximal end (17) and a distal end (12),
with at least one tool (13) arranged on the distal end (12),
with a handle (18) arranged on the proximal end (17),
with at least one actuating device (24) arranged on the handle (18),
with at least one wire (30) which extends through the interior of the shaft (11) and is movable therein in order to transmit a movement of the actuating device (24) to the tool (13),
with at least one sealing element (34) which is arranged in the interior of the tube forming the shaft (11) and closes the entire cross-section of the shaft (11) so that the wire (30) extends through the sealing element (34) in a linearly movable fashion, **characterised in that** the sealing element (34) in the shaft (11) is formed by first forming to a length which is greater than the internal diameter and shorter than the length of the shaft (11).

2. Instrument according to claim 1, **characterised in that** the tool (13) comprises at least one linearly movable or pivotably movable part (14) which is connected to the transmission means (29, 29a).

3. Instrument according to claim 1 or 2, **characterised in that** the transmission means (29, 29a, 29b) is a wire (30) or a thin rod (31) made of metal or plastic.

4. Instrument according to any of the preceding claims, **characterised in that** the transmission means (29, 29a, 29b) is electrically conductive.

5. Instrument according to any of the preceding claims, **characterised in that** the tool (13) has two or more movable parts (14, 15) connected to two or more transmission means (29, 29a) which extend through the shaft (11) and through the sealing element (34), wherein the transmission means (29, 29a) are movable relative to each other.

6. Instrument according to claim 1, **characterised in that** at least the portion of the transmission means (29, 29a, 29b) which extends through the sealing element (34) is formed straight.

7. Instrument according to any of the preceding claims, **characterised in that** the sealing element (34) consists of a hydrophobic material.

8. Instrument according to any of the preceding claims, **characterised in that** the sealing element (34) has a length measured in the shaft longitudinal direction which exceeds by a multiple the diameter of the sealing element (34) and/or which is the same as or less than the length of the shaft (11), wherein the shaft longitudinal direction is established by shaft centre axis (36).

9. Instrument according to any of the preceding claims, **characterised in that** the sealing element (34) is arranged at the distal end (12) of the shaft (11), at the proximal end (17) of the shaft (11), and/or in between.

10. Instrument according to any of the preceding claims, **characterised in that** the sealing element (34) is held in the shaft (11) so as to be immovable relative to the shaft (11).

11. Instrument according to any of the preceding claims, **characterised in that** the sealing element (34) is held in the shaft (11) by force fit, friction fit, material fit and/or form fit.

## Revendications

1. Instrument chirurgical (10), en particulier instrument endoscopique,
comprenant une tige (11) allongée, qui est un tube présentant un espace intérieur et présentant une extrémité proximale (17) et une extrémité distale (12),
comprenant au moins un outil (13) qui est disposé sur l'extrémité distale (12),
comprenant une poignée (18) qui est disposée sur l'extrémité proximale (17),
comprenant au moins un dispositif d'actionnement (24) qui est placé sur la poignée (18),
comprenant au moins un fil métallique (30) qui s'étend à travers l'espace intérieur de la tige (11) et est déplaçable dans celui-ci, afin de transmettre un mouvement du dispositif d'actionnement (24) à l'outil (13),
comprenant au moins un élément d'étanchéité (34) qui est disposé dans l'espace intérieur du tube formant la tige (11) et obture la totalité de la section transversale de la tige (11), de sorte que le fil métallique (30) s'étend avec possibilité de déplacement longitudinal à travers l'élément d'étanchéité (34),
**caractérisé en ce que** l'élément d'étanchéité (34) est réalisé dans la tige (11) par des formes primitives sur une longueur qui est supérieure au diamètre intérieur et inférieure à la longueur de la tige (11).

2. Instrument selon la revendication 1, **caractérisé en ce que** l'outil (13) comprend au moins un élément (14) susceptible d'effectuer un mouvement linéaire ou pivotant, qui est relié au moyen de transmission (29, 29a).

3. Instrument selon la revendication 1 ou 2, **caractérisé en ce que** le moyen de transmission (29, 29a, 29b) est un fil métallique (30) ou une tige (31) mince en métal ou matière plastique.

4. Instrument selon l'une des revendications précédentes, **caractérisé en ce que** le moyen de transmission (29, 29a, 29b) est conducteur électrique.

5. Instrument selon l'une des revendications précédentes, **caractérisé en ce que** l'outil (13) présente deux ou plusieurs éléments (14, 15) mobiles, qui sont reliés à deux ou plusieurs moyens de transmission (29, 29a) qui s'étendent à travers la tige (11) et à travers l'élément d'étanchéité (34), les moyens de transmission (29, 29a) pouvant être déplacés l'un par rapport à l'autre.

6. Instrument selon la revendication 1, **caractérisé en ce qu'**au moins la portion du moyen de transmission (29, 29a, 29b) s'étendant à travers l'élément d'étanchéité (34) est réalisée sous une forme rectiligne.

7. Instrument selon l'une des revendications précédentes, **caractérisé en ce que** l'élément d'étanchéité (34) est constitué d'un matériau hydrophobe.

8. Instrument selon l'une des revendications précédentes, **caractérisé en ce que** l'élément d'étanchéité (34) présente une longueur, à mesurer dans le sens longitudinal de la tige, qui correspond à plusieurs fois le diamètre de l'élément d'étanchéité (34) et/ou qui est égale ou inférieure à la longueur de la tige (11), la direction longitudinale de la tige étant définie par un axe médian de tige (36).

9. Instrument selon l'une des revendications précédentes, **caractérisé en ce que** l'élément d'étanchéité (34) est disposé à l'extrémité distale (12) de la tige (11), à l'extrémité proximale (17) de la tige (11) et/ou entre les deux.

10. Instrument selon l'une des revendications précédentes, **caractérisé en ce que** l'élément d'étanchéité (34) est maintenu dans la tige (11), de manière à ne pas pouvoir être déplacé par rapport à la tige (11).

11. Instrument selon l'une des revendications précédentes, **caractérisé en ce que** l'élément d'étanchéité (34) est maintenu dans la tige (11) par adhérence, par friction, par matière et/ou par liaison mécanique.
